# EUROPEAN PATENT SPECIFICATION

(11) **EP 0 908 441 B1**
(45) Date of publication and mention of the grant of the patent: **28.08.2002**
(21) Application number: 98307913.8
(22) Date of filing: 29.09.1998
(51) Int. Cl.: C07C 45/51, C07C 47/21

(54) **Process for producing 7-octen-1-al**
Verfahren zur Herstellung von 7-Octen-1-al
Procédé pour la préparation de 7-octène-1-al

(30) Priority: 30.09.1997 JP 26623297
(43) Date of publication of application: 14.04.1999
(73) Proprietor: KURARAY CO., LTD., Kurashiki-City Okayama Prefecture 710 (JP)
(72) Inventor: Tsuda, Tomoyasu, Kurashiki-City, Oakayama-Pref., 710-8622 (JP); Tokitoh, Yasuo, c/o Kuraray Co., Ltd., Kitakanbara-gun, Niigata-Pref., 959-2653 (JP); Watanabe, Kazunori, Kurashiki-City, Oakayama-Pref., 710-8622 (JP); Hori, Takashi, Kurashiki-City, Okayama-Pref., 710-0801 (JP)
(74) Representative: Nachshen, Neil Jacob

(56) References cited:
- EP-A- 0 069 339
- GB-A- 1 265 044
- US-A- 4 463 196
- CHEMICAL ABSTRACTS, vol. 114, no. 7, 18 February 1991 Columbus, Ohio, US; abstract no. 061540, TOKITO Y ET AL: "Preparation of 7-octen-1-al" XP000182933 & JP 02 218638 A (KURARAY CO., LTD.;JAPAN) 31 August 1990
- CHEMICAL ABSTRACTS, vol. 122, no. 11, 13 March 1995 Columbus, Ohio, US; abstract no. 132583, SAITO H ET AL: "Copper catalysts for (de)hydrogenation and their use in preparation of octenal" XP002088126 & JP 06 210173 A (KURARAY CO;JAPAN) 2 August 1994

## Description

The present invention relates to a process for producing 7-octen-al by isomerising 2,7-octadien-1-ol. 7-Octen-1-al, which contains a terminal double bond and an aldehyde group with high reactivity, is very useful as a starting material for various industrial chemicals.

For instance, 7-octen-1-al is converted into 1,9-nonanedial by hydroformylation, which can give various starting materials for synthesising polymers, such as 1,9-nonanediol, azelaic acid and 1,9-nonanediamine. Also, 7-octen-1-al can be converted, by oxidation with oxygen in the presence of a catalyst such as a cobalt salt, a manganese salt, a nickel salt, a copper salt or an iron salt, into 7-octenic acid. Furthermore, 7-octen-1-al can be converted by reduction into 7-octen-1-ol or 1-octanol, or can be converted, by reduction of the double bond and oxidation of the aldehyde group, into capric acid.

### Description of the Prior Art

GB 1,265,044 discloses a process for producing an aldehyde which comprises isomerising an allylic alcohol in the presence of hydrogen and a hydrogenation-dehydrogenation catalyst at elevated temperature. GB 1,265,044 neither discloses nor suggests a process for producing 7-octenl-al by isomerisation of 2,7-octadien-1-ol or the effect of adding hydrogen to suppress the byproduction of 2,7-octadien-1-al and other high boiling compounds.

Japanese Patent Publication No. 60378/1987 and Japanese Patent Application Laid-open No. 118535/1983 disclose a process for producing 7-octen-1-al, which comprises isomerising 2,7-octadien-1-ol in the presence of a catalyst selected from the group consisting of copper catalysts and chromium catalysts. Japanese Patent Application Laid-open No. 218638/1990 (Patent No. 2565561) discloses a process for producing 7-octen-1-al, which comprises isomerising, at a temperature of 180 to 250°C, 2,7-octadien-1-ol in the presence of at least one compound selected from the group consisting of n-octanol, 3-octanol and 7-octen-1-ol in an amount of 50 to 200% by weight based on the weight of 2,7-octadien-1-ol and in the presence of metal oxide catalyst containing at least 2 metals selected from the group consisting of copper, chromium and zinc.

The process described in Japanese Patent Publication No. 60378/1987 and Japanese Patent Application Laid-open No. 118535/1983 has the problems that there is inevitably by produced 2,7-octadien-1-al from which it is difficult to separate by distillation the product 7-octen-1-al and that the byproduced 2,7-octadien-1-al from which it is difficult to separate by distillation the product 7-octen-1-al and that the byproduced 2,7-octadien-1-al, which is a catalyst poison for hydroformylation, decreases, on hydroformylation of the obtained 7-octen-1-al to form 1,9-nonanedial, the yield of the 1,9-nonanedial. This fact is clear from the description in Japanese Patent Application Laid-open No. 218638/1990 that when the reaction is conducted in accordance with the procedure given in Examples of Japanese Patent Publication No. 60378/1987 with repeated use of the same catalyst, there tends to occur a gradual increase of byproduced 2,7-ocatadien-1-al. The process described in Japanese Patent Application Laid-open No. 218638/1990 decreases the amount of 2,7-octadien-1-al byproduced, to a low level. Example 2 shows only 0.7%, which is the largest figure in the Examples, of the by-product after 7-hour reaction. However, with this process, n-octanol and 3-octanol, which are used as sources for generating hydrogen, are necessarily converted, by dehydrogenation during the reaction, into n-octylaldehyde (boiling point: 173°C/0.101MPa (760 mmHg)) and 3-octanone (boiling point: 168°C/0.101MPa (760 mmHg)) respectively each having a boiling point very close to that (174°C/0.101MPa (760 mmHg)) of the desired product 7-octen-1-al. As a result, the process described in Japanese Patent Application Laid-open No. 218638/1990 has the problem that it is very difficult to separate the desired product from these by-products by distillation.

In order to solve the above problem of the process described in Japanese Patent Application Laid-open No.218638/1990, it is necessary to develop a process which does not use n-octanol, 3-octanol or 7-octen-1-ol as sources for generating hydrogen. The present inventors have therefore studied in detail the mechanism of formation of 7-octen-1-al from 2,7-octadien-1-ol. They have found that the reaction is a formal isomerisation reaction which comprises dehydrogenation of the allyl alcohol part of 2,7-octadien-1-ol to form 2,7-octadien-1-al and the succeeding hydrogenation of the carbon-carbon double bond conjugating with the aldehyde group to form 7-octen-1-al. Thus, the byproduction of 2,7-octadien-1-al in this reaction is caused by the fact that the hydrogen having formed on dehydrogenation is consumed, not only for hydrogenation to form 7-octen-1-al, but for various byproduction, thereby causing a part of the intermediate product 2,7-octadien-1-al to remain in the reaction zone. Indeed, the present inventors conducted isomerization in accordance with the procedures given in Comparative Examples 1, 3, 4 and 5 in the instant specification to confirm that 2,7-octadien-1-al was byproduced in a large amount. The present inventors have also found that, on a long-period isomerization, as shown in Comparative Example 6 of the instant specification, the amount of 2,7-octadien-1-al byproduced increases and, at the same time, the conversion of 2,7-octadien-1-ol decreases, so that the selectivity to 7-octen-1-al decreases. In summary, the serious problem found is that 2,7-octadien-1-al decreases the catalytic activity for the isomerization of the present invention.

Furthermore, where the reaction is conducted by a reaction-distillation method, which is regarded as a particularly preferred embodiment in Japanese Patent Publication No. 60378/1987, Japanese Patent Application · Laid-open No. 118535/1983 and Japanese Patent Application Laid-open No. 218638/1990, there occurs another problem that there inevitably accumulates high-boiling byproducts originating from the aldehydes formed by the reaction, which leads to a decrease in the yield.

It is therefore important, in order to conduct the reaction operation over a long period of time, to suppress not only the formation of 2,7-octadien-1-al that decreases the catalytic activity but also that of high-boiling byproducts. As described above, the mechanism involved in the formation of 7-octen-1-al from 2,7-octadien-1-ol is a formal isomerization which comprises dehydrogenation of the allyl alcohol part of 2,7-octadien-1-ol to form 2,7-octadien-1-al and the succeeding hydrogenation of the carbon-carbon double bond conjugating with the aldehyde group to form 7-octen-1-al. The byproduction of 2,7-octadien-1-al results from the fact that the hydrogen formed on the above dehydrogenation is consumed, not only for the hydrogenation to 7-octen-1-al, but for various side reactions, which renders insufficient the hydrogenation of 2,7-octadien-1-al and thus causing this aldehyde to remain in the reaction zone. Hydrogenation of the remaining 2,7-octadien-1-al by adding hydrogen, which is inexpensive, to the reaction zone could reduce the content of 2,7-octadien-1-al in the final reaction mixture. From this viewpoint the present inventors decided to study the hydrogenation conditions in detail. The specification of Japanese Patent Publication No. 60378/1987 describes "The isomerization reaction is preferably carried out in an atmosphere of a gas which is inert under the reaction condition. The whole or part of the inert gas may be replaced with hydrogen gas. When the reaction is carried out in the co-presence of hydrogen, the hydrogen partial pressure is preferably kept below 1.01 MPa (10 atmospheres). At a hydrogen partial pressure exceeding 1.01 MPa (10 atmospheres), the hydrogenation reaction prevails, unfavorably causing decrease in the selectivity toward 7-octen-1-al." and the same description is disclosed in the specification of Japanese Patent Application Laid-open No. 118535/1983. However, the specification of Japanese Patent Publication No. 60378/1987 only gives, in its Examples 2 and 4, the results that the ratios of isomerization to 7-octen-1-al under a hydrogen atmosphere were 43% and 73%, respectively, and gives no description about the amount of hydrogen added. The specification of Japanese Patent Application Laid-open No. 118535/1983 only gives, in its Examples 4 and 6, the results that the selectivity toward 7-octen-1-al under the flow of hydrogen gas were 92% and 79%, respectively. The present inventors have studied this reaction and found the selectivity toward 7-octen-1-al is low, that is, the byproduction of the other hydrogenation products prevails in this reaction, and the activity of the catalyst decreases as the reaction proceeds, shown in Comparative Example 7 of this specification. All the catalysts disclosed in these prior art references are known catalysts used for hydrogenation. It is therefore expected that addition of hydrogen to the reaction zone causes various hydrogenation side reactions and, in some cases, may cause the product 7-octen-1-al to be further hydrogenated, thereby decreasing the selectivity to 7-octen-1-al. However, these prior art references give no concrete description about the effect of the addition of hydrogen to suppress byproduction of 2,7-octadien-1-al.

### SUMMARY OF THE INVENTION

As a result of an extensive study on the method and amount used and the like of hydrogen addition, the present inventors have found, surprisingly, that it is possible, by conducting the reaction in the presence of a copper catalyst, while controlling the ratio between the amounts of 2,7-octadien-1-ol and hydrogen within a specific range, to suppress the byproduction of 2,7-octadien-1-al without decreasing the selectivity to the product 7-octen-1-al and that conducting the reaction in the gaseous phase, which facilitates rapid removal of the product from the reaction zone, can suppress formation of high-boiling byproducts, whereby stable reaction operation becomes possible over a long period of time.

The present invention seeks to provide a process for producing 7-octen-1-al by isomerization of 2,7-octadien-1-ol, which comprises feeding 2,7-octadien-1-ol and hydrogen to a reaction zone in the presence of a copper catalyst, while controlling the molar ratio of 2,7-octadien-1-ol to hydrogen within a range of 99/1 to 75/25, and effecting isomerization in the gaseous phase.

### DETAILED DESCRIPTION OF THE PREFERRED EMBODIMENTS

In the present invention, the amount of hydrogen to be fed together with 2,7-octadien-1-ol is, in terms of the molar ratio of 2,7-octadien-1-ol to hydrogen, within a range of 99/1 to 75/25. This range corresponds to 1 to 25 mole % of hydrogen in the resulting mixture of 2,7-octadien-1-ol and hydrogen. The molar ratio of 2,7-octadien-1-ol to hydrogen is preferably within a range of 97/3 to 80/20. If hydrogen is used in a molar ratio of 2,7-octadiene to hydrogen exceeding 99/1, i.e. in less than 1 mole % of hydrogen in the mixture consisting of the two components, there will only be a small effect of suppressing byproduction of 2,7-octadien-1-al. On the other hand, use of hydrogen in a molar ratio of 2,7-octadien-1-ol to hydrogen of less than 75/25, i.e. in an amount exceeding 25 mole % of hydrogen in the mixture consisting of the two components decreases the selectivity to 7-octen-1-al.

Examples of suitable copper catalysts used in the process of the present invention include reduced copper, Raney copper, copper-zinc oxide, copper-chromium oxide, copper-aluminum oxide, copper-iron-aluminum oxide, copper-zinc-aluminum oxide and copper-zinc-titanium oxide. These metal catalysts, which are commercially produced and readily available, can also be prepared in accordance with the processes described in, for example, "Shokubai Kogaku Koza 10; List of Element-Based Catalysts 365-367 ", issued on February 25, 1967 from Chijin Shokan Co., Ltd. These catalysts may be partially modified with other metal components selected from tungsten, molybdenum, rhenium, zirconium, manganese, titanium, barium or the like. These catalysts may also be used in a form carried on a carrier such as alumina, silica or diatomaceous earth. These catalysts may optionally have their surfaces pretreated with an alkali metal and/or an alkali earth metal before use. These catalysts may be used singly or in combination of 2 or more.

It is preferable to activate the catalyst by treating with hydrogen before use, which ensures development of higher catalytic activity.

In the process of the present invention, on feeding 2,7-octadien-1-ol and hydrogen, it is optional to feed together with the 2,7-octadien-1-ol, a primary or secondary alcohol represented by the general formula (I)

R¹R²CH-OH (I)

wherein R¹ and R² each independently represents a hydrogen atom, or an alkyl, alkenyl, aryl or cycloalkyl group which may be substituted; or R¹ and R² together represent a cycloalkyl group formed by binding R¹ and R², which may be substituted. The alcohol is fed into the reaction zone, prior to or simultaneously with the 2,7-octadien-1-ol, through the same or a different inlet.

More preferably, R¹ and R² in the formula (I) each independently represents a hydrogen atom, a linear or branched alkyl having 1 to 10 carbon atoms, or an alkenyl, aryl or cycloalkyl group which may be substituted with a lower alkyl group having 1 to 3 carbon atoms or phenyl group; or R¹ and R² together represent a cycloalkyl group which may be substituted with a lower alkyl group having 1 to 3 carbon group.

Examples of the primary or secondary alcohol which may be used in the present invention are saturated primary alcohols, e.g. methanol, ethanol, n-propanol, n-butanol, isobutyl alcohol, n-pentanol, isopentyl alcohol, 2-methyl-1-butanol, neopentyl alcohol, n-hexanol, 2-methyl-1-pentanol, 2-ethyl-1-butanol, n-heptanol, n-octanol, 2-ethyl-1-hexanol, n-nonanol, 3,5,5-trimethyl-1-hexanol and n-decanol; unsaturated primary alcohols, e.g. allyl alcohol, methallyl alcohol, crotyl alcohol, 4-penten-1-ol, 3-methyl-3-buten-1-ol, 3-methyl-2-buten-1-ol, 5-hexen-1-ol and 7-octen-1-ol; aralkyl primary alcohols, e.g. benzyl alcohol; linear secondary alcohol, e.g. 2-propanol, 2-butanol, 2-pentanol, 3-pentanol, 4-methyl-2-pentanol, 2-heptanol, 2-octanol, 3-octanol and diisobutylcarbinol; and cyclic secondary alcohols, e.g. cyclopentanol, cyclohexanol and methyl-cyclohexanol. These alcohols may be used singly or in combination of 2 or more.

The process of the present invention can be carried out under atmospheric pressure, under a pressure or under a reduced pressure. As the reaction apparatus, for example the usual fixed bed flow-system reaction apparatus can be used. Use of the flow-system reaction facilitates rapid removal of the product from the reaction zone, thereby suppressing formation of high boiling byproducts from aldehyde group-containing compounds.

In carrying out the process of the present invention, it is preferable, when mixing 2,7-octadien-1-ol and hydrogen and, if desired, a primary or secondary alohol, with an inert carrier gas, to contact the mixed gas with a copper catalyst. However, the use of such an inert carrier gas is not essential in the process of the present invention. Thus, 2,7-octadien-1-ol may be simply mixed with hydrogen and, if desired, a primary or secondary alcohol and the obtained mixed gas may be used as the gaseous starting material. Nitrogen is preferably used as the inert carrier gas when used in the process of the present invention.

The reaction temperature is preferably in a range of 100 to 260° C, more preferably in a range of 160 to 240° C. At a temperature lower than 100° C, the reaction tends to proceed very slowly. On the other hand, at a temperature higher than 260° C, there occurs sintering of the catalyst used, so that the catalyst is degraded with metallic copper precipitating on the surface, thereby decreasing both the selectivity and catalyst life.

The process of the present invention can be carried out over a wide range of the liquid hourly space velocity (LHSV) of 2,7-octadien-1-ol. The preferred range of LHSV is from 0.01 to 20 hr⁻¹, and a range of 0.5 to 10 hr⁻¹ is more preferred. An LHSV of less than 0.01 hr⁻¹, which leads to a very low productivity of 7-octen-1-al, cannot be used on a commercial scale. On the other hand, an LHSV exceeding 20 hr⁻¹ decreases the conversion of 2,7-octadien-1-ol.

The 7-octen-1-al that forms by the reaction can be separated from the reaction mixture by the usual distillation.

Other features of the invention will become apparent in the course of the following detailed descriptions of exemplary embodiments which are given for illustration of the invention and are not intended to be limiting thereof. In the Examples and Comparative Examples that follow, the following abbreviations are used.
- GHSV:: gas hourly space velocity (hr⁻¹); defined as:
[Volume per hour of feed gas passing the total volume of catalyst]/[total volume of catalyst]
- LHSV:: liquid hourly space velocity (hr⁻¹); defined as:
[Volume per hour of feed liquid passing the total volume of catalyst]/[total volume of catalyst]

There were used following reaction apparatus, as well as the following quantitative analysis, catalyst activation treatment and reaction procedure.

### Reaction apparatus

A quartz glass tube having an inner diameter of 24 mm and a total length of 720 mm and equipped in its inside a thermometer sheath tube was packed with 25 ml of a copper catalyst. The copper catalysts used were all molded pellets and had a length of catalyst layer of about 65 mm. The bottom part of the tube beneath the catalyst layer was packed with glass beads having an average particle diameter of 5 mm, and on the top thereof with the same glass beads over a height of about 150 mm, which constituted a pre-heating zone. The catalyst and glass beads portions were heated in a cylindrical electric oven and the temperature of the catalyst layer, which was reaction temperature, was measured and adjusted to a prescribed temperature. The bottom part of the reaction tube was fitted with a cooling device, sampler, reservoir and cold trap, so that the distilled reaction mixture was recoverable. A metering feed pump was used to feed 2,7-octadien-1-ol at a prescribed rate and a gas mixer capable of adjusting the composition and flow rate was used to feed nitrogen and hydrogen.

### Analysis and quantitative analysis of reaction mixture

The distillate was sampled at a prescribed interval and subjected to gas chromatography by using capillary columns (DB-WAX, 30 m, diameter: 0.25 mm, film: 0.25 µ m) made by J & W Scientific Company and with Triglyme as an internal standard substance.

### Analytical conditions

Injection temperature and detection temperature: 280° C
Temperature elevation program: 100° C (kept for 2 minutes) → 6° C/min → 240 C (kept for 5 minutes)

### Activation of catalyst by treating with hydrogen

After the inside of the reaction apparatus packed with the catalyst had been sufficiently replaced by nitrogen, the catalyst layer was gradually heated while nitrogen was fed at GHSV = 300 hr⁻¹. After the temperature of the catalyst layer had exceeded 120° C, a nitrogen/hydrogen mixed gas containing 3% of hydrogen was introduced. The mixing ratio of hydrogen was gradually increased, while care was taken to keep the catalyst layer temperature from exceeding 220 ° C to prevent vigorous heat generation. When heat generation was no longer observed, the temperature of the catalyst layer was maintained at 200 to 220° C for 4 to 5 hours, while only hydrogen was fed at GHSV = 300 hr⁻¹.

### Reaction procedure

After completion of the activation treatment of catalyst, the temperature of the reaction layer was, while hydrogen was continuously introduced, adjusted to about 20° C lower than the intended reaction temperature. The feed gas was then changed to a mixed gas having a prescribed nitrogen/hydrogen ratio, which was fed at a prescribed GHSV. Thereafter, 2,7-octadien-1-ol was fed at a prescribed LHSV and the heating was adjusted to achieve the desired temperature. While the reaction temperature was kept constant, the distillate was sampled at a prescribed time and subjected to analysis.

### Examples 1 through 5 and Comparative Examples 1 through 5

Reactions were carried out with various copper catalysts, with the LHSV of 2,7-octadien-1-ol set at 1.5 hr⁻¹ and with various reaction temperatures and compositions of mixed nitrogen/hydrogen gases. Table 1 shows the compositions of the copper catalysts used and Table 2 the reaction results after 2-hour reaction.

**Table 1**

| Copper Catalyst Used | | | | |
|---|---|---|---|---|
| (all made by Nikki Kagaku Co., Ltd.) | | | | |

| Code | Composition (% by weight) | | | |
|---|---|---|---|---|
| N202D | CuO: 38.5 | Cr₂O₃: 35.6 | MnO₂: 2.0 | |
| N202E | CuO: 39.8 | Cr₂O₃: 41.1 | MnO₂: 1.9 | BaO: 1.7 |
| E26L | Cu : 23.7 | Fe : 20.9 | Al : 18.6 | Zn : 1.3 |
| N211 | CuO: 49.0 | ZnO : 43.6 | | |

### Average diameter of pellets: φ5x5 (N202D, N202E, E26L) φ6x6 (N211)

The results of Examples 1 through 5 and Comparative Examples 1 through 5 clearly show that, in the reactions producing 7-octen-1-al with various copper catalysts, co-presence of hydrogen reduces the amount of 2,7-octadien-1-al byproduced. It is also understood from the results of Comparative Example 2, that the amount of the co-present hydrogen exceeding the range specified in the instant specification reduces the selectivity to the desired 7-octen-1-al.

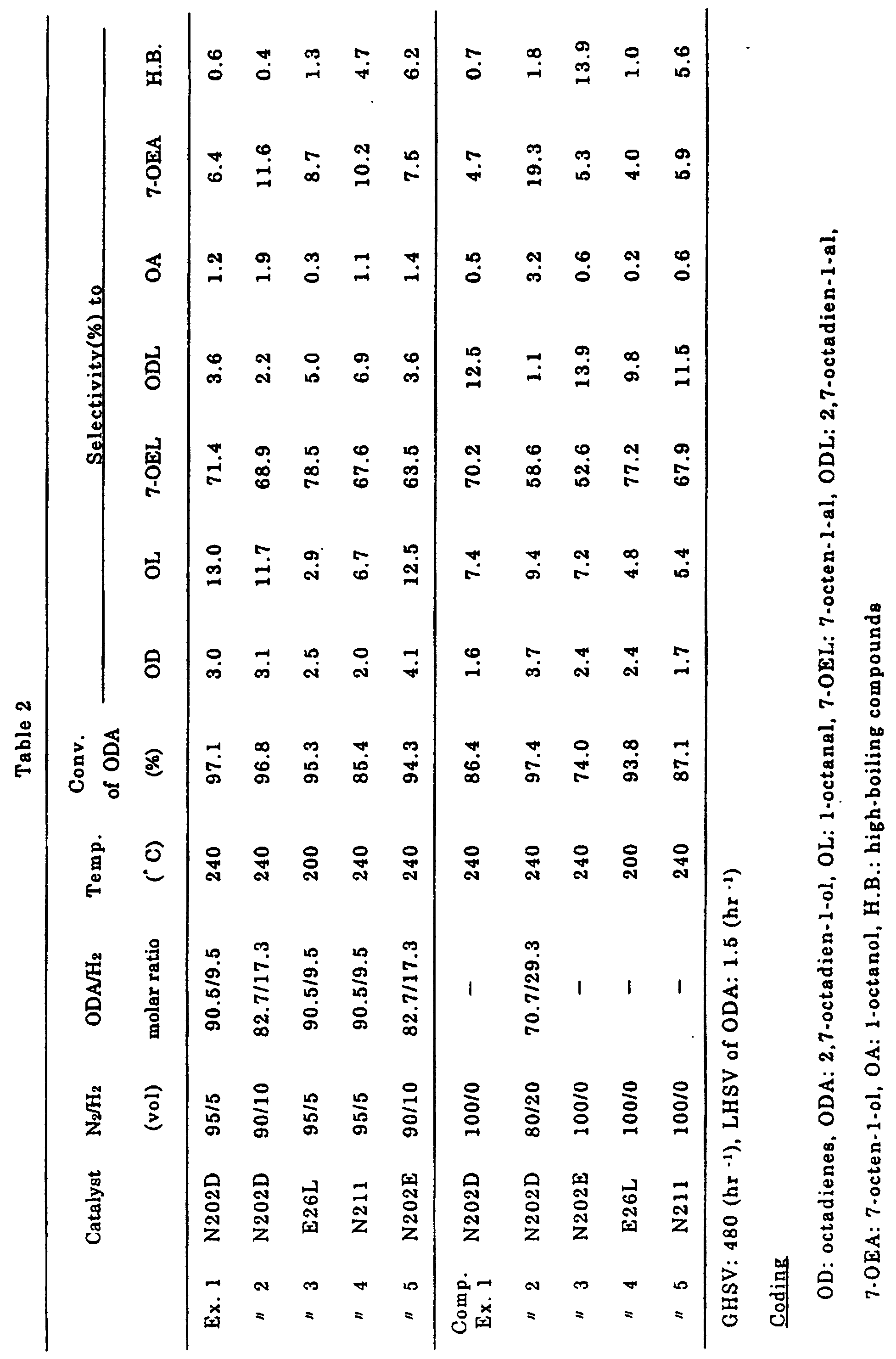

### Example 6

Reaction was carried out with a catalyst of copper-iron-aluminum catalyst (E26L; made by Nikki Kagaku Co., Ltd.) at a reaction temperature of 220° C and by feeding 2,7-octadien-1-ol at an LHSV of 1.5 hr⁻¹ and a 95/5 by volume nitrogen/hydrogen mixed gas at a GHSV of 480hr⁻¹. The above volume ratio corresponds to a molar ratio of 2,7-octadien-1-ol to hydrogen of 90.5/9.5. The change with time of the reaction results is shown in Table 3. It is understood that good reaction results were obtained even 200 hours after continuation of the reaction operation.

**Table 3**

| Reaction time | Conversion of 2,7-octadien-1-ol | Selectivity to | |
|---|---|---|---|
| | | 7-octen-1-al | 2,7-octadien-1-al |
| (hr) | (%) | (%) | (%) |
| 5 | 99.0 | 76.8 | 3.3 |
| 50 | 95.8 | 78.2 | 3.1 |
| 100 | 96.1 | 77.4 | 3.3 |
| 200 | 95.5 | 76.7 | 3.7 |

### Comparative Example 6

Reaction was carried out with a catalyst of copper-iron-aluminum catalyst (E26L; made by Nikki Kagaku Co., Ltd.) at a reaction temperature of 220° C and by feeding 2,7-octadien-1-ol at an LHSV of 1.5hr⁻¹ and nitrogen gas at a GHSV of 480 hr⁻¹. The change with time of the reaction results is shown in Table 4. It is understood that the conditions employed here without presence of hydrogen led to a significant byproduction of 2,7-octadien-1-al and a decrease in both of the conversion and the selectivity.

**Table 4**

| Reaction time | Conversion of 2,7-octadien-1-ol | Selectivity to | |
|---|---|---|---|
| | | 7-octen-1-al | 2,7-octadien-1-al |
| (hr) | (%) | (%) | (%) |
| 5 | 93.6 | 76.0 | 10.0 |
| 50 | 87.5 | 73.6 | 11.5 |
| 100 | 76.6 | 72.2 | 13.1 |

### Example 7

Reaction was carried out with a catalyst of copper-iron-aluminum catalyst (E26L; made by Nikki Kagaku Co., Ltd.) at a reaction temperature of 220° C and by feeding a 90/10 by moles 2,7-octadien-1-ol/7-octen-1-ol mixed gas at an LHSV of 1.7 hr⁻¹ and a 98/2 by volume nitrogen/hydrogen mixed gas at a GHSV of 480 hr⁻¹. The above volume ratio corresponds to a molar ratio of 2,7-octadien-1-ol to hydrogen of 96/4. The change with time of the reaction results is shown in Table 5. It is understood that no change occurred at all in the catalytic activity even after a time elapse of 100 hours.

**Table 5**

| Reaction time | Conversion of 2,7-octadien-1-ol | Selectivity to | |
|---|---|---|---|
| | | 7-octen-1-al | 2,7-octadien-1-al |
| (hr) | (%) | (%) | (%) |
| 5 | 98.7 | 85.6 | 0.6 |
| 50 | 99.0 | 85.2 | 0.7 |
| 100 | 98.5 | 85.1 | 0.8 |

### Comparative Example 7

A 100-ml three-necked flask, which was connected to a distillation apparatus, equipped with an electromagnetic stirrer, an inlet for liquid and an another inlet for gas, was charged with 30 ml of 2,7-octadien-1-ol and 2.0 g of Raney-copper (made by Kawaken Fine Chemical Co., Ltd.) and was immersed in an oil-bath, whose temperature was maintained at 205° C. With vigorous stirring of the mixture in this flask, hydrogen gas was introduced into the flask at the rate of 30 l/hr, and 2,7-octadien-1-ol was continuously introduced at the rate of 170 ml/hr. As the distillate was collected at the rate of 170 ml/hr, the volume of the content in the reaction flask was controlled to maintain about 30 ml. After stating the reaction, the distillate was periodically analyzed by the same method described above. The result is shown in Table 6. It is understood that the selectivity toward 7-octen-1-al is low and the activity of the catalyst decreases as the reaction proceeds.

**Table 6**

| Reaction time | Conversion of 2,7-octadien-1-ol | Selectivity to | |
|---|---|---|---|
| | | 7-octen-1-al | 2,7-octadien-1-al |
| (hr) | (%) | (%) | (%) |
| 5 | 93.1 | 9.1 | 0.2 |
| 10 | 86.8 | 8.9 | 0.5 |
| 20 | 59.2 | 8.5 | 1.1 |

In Tables 2 through 6, the selectivities were calculated based on the starting material ODA.

Obviously, numerous variations and modifications of the invention are possible in light of the above teachings. It is therefore to be understood that within the scope of the appended claims, the invention may be practiced otherwise than as specifically described herein.

## Claims

1. A process for producing 7-octen-1-al by isomerization of 2,7-octadien-1-ol, which comprises feeding 2,7-octadien-1-ol and hydrogen to a reaction zone in the presence of a copper catalyst, the molar ratio of 2,7-octadien-1-ol to hydrogen being within a range of 99/1 to 75/25, and effecting isomerization in the gaseous phase.

2. The process according to claim 1, wherein the molar ratio of 2,7-octadien-1-ol to hydrogen is within the range of 97/3 to 80/20.

3. The process according to either claim 1 or 2, wherein 2,7-octadien-1-ol and hydrogen are mixed with an inert carrier gas while or prior to feeding to the reaction zone.

4. The process according to claim 3, wherein said inert carrier gas is nitrogen.

5. The process according to any of claims 1-4, wherein said isomerization is effected at a temperature in a range of 100 to 260°C.

6. The process according to any of claims 1-5, wherein said 2,7-octadien-1-ol is fed to the reaction zone at an LHSV (liquid hourly space velocity) of 0.01 to 20hr⁻¹.

7. A process according to any preceding claim, wherein a primary or secondary alcohol of formula (I)
R¹R²CH-OH (I)
wherein R¹ and R² each independently represents a hydrogen atom, or an alkyl, alkenyl, aryl or cycloalkyl group which may be substituted; or R¹ and R² together represent a cycloalkyl group formed by binding R¹ and R², which may be substituted, is fed into the reaction zone with the 2,7-octadien-1-ol.

8. A process according to claim 7, wherein the R¹ and R² in the formula (I) each independently represents a hydrogen atom, a linear or branched alkyl having 1 to 10 carbon atoms, or an alkenyl, aryl or cycloalkyl group which may be substituted with a lower alkyl group having 1 to 3 carbon atoms or phenyl group; or R¹ and R² together represent a cycloalkyl group which may be substituted with a lower alkyl group having 1 to 3 carbon group.

9. A process according to claim 7 or 8, wherein the alcohol is fed into the reaction zone, prior to or simultaneously with the 2,7-octadien-1-ol.

## Patentansprüche

1. Verfahren zur Herstellung von 7-Octen-1-al durch Isomerisierung von 2,7-Octadien-1-ol, welches das Beschicken von 2,7-Octadien-1-ol und Wasserstoff in eine Reaktionszone in Gegenwart eines Kupferkatalysators, wobei das molare Verhältnis von 2,7-Octadien-1-ol zu Wasserstoff in einem Bereich von 99/1 bis 75/25 liegt, und das Bewirken der Isomerisierung in der Gasphase umfaßt.

2. Verfahren nach Anspruch 1, wobei das molare Verhältnis von 2,7-Octadien-1-ol zu Wasserstoff in dem Bereich von 97/3 bis 80/20 liegt.

3. Verfahren nach Anspruch 1 oder 2, worin 2,7-Octadien-1-ol und Wasserstoff mit einem inerten Trägergas während oder vor dem Beschicken in die Reaktionszone gemischt werden.

4. Verfahren nach Anspruch 3, wobei das inerte Trägergas Stickstoff ist.

5. Verfahren nach einem der Ansprüche 1 bis 4, worin die Isomerisierung bei einer Temperatur in einem Bereich von 100 bis 260°C bewirkt wird.

6. Verfahren nach einem der Ansprüche 1 bis 5, worin das 2,7-Octadien-1-ol in die Reaktionszone mit einer LHSV (stündliche Raumgeschwindigkeit der Flüssigkeit) von 0,01 bis 20 h⁻¹ beschickt wird.

7. Verfahren nach einem vorhergehenden Anspruch, worin ein primärer oder sekundärer Alkohol der Formel (I)
R¹R²CH-OH (I)
worin die Reste R¹ und R² jeweils unabhängig voneinander ein Wasserstoffatom oder einen Alkyl-, Alkenyl-, Aryl- oder Cycloalkylrest, der substituiert sein kann, darstellen oder die Reste R¹ und R² miteinander einen Cycloalkylrest, gebildet durch Binden von R¹ und R², der substituiert sein kann, darstellen, in die Reaktionszone mit dem 2,7-Octadien-1-ol beschickt wird.

8. Verfahren nach Anspruch 7, wobei die Reste R¹ und R² in der Formel (I) jeweils unabhängig voneinander ein Wasserstoffatom, ein lineares oder verzweigtes Alkyl mit 1 bis 10 Kohlenstoffatomen oder einen Alkenyl-, Aryl- oder Cycloalkylrest, die mit einem Niederalkylrest mit 1 bis 3 Kohlenstoffatomen oder einer Phenylgruppe substituiert sein können, darstellen oder die Reste R¹ und R² miteinander einen Cycloalkylrest, der mit einem Niederalkylrest mit 1 bis 3 Kohlenstoffatomen substituiert sein kann, darstellen.

9. Verfahren nach Anspruch 7 oder 8, worin der Alkohol in die Reaktionszone vor oder gleichzeitig mit dem 2,7-Octadien-1-ol beschickt wird.

## Revendications

1. Procédé pour la préparation de 7-octèn-1-al par isomérisation du 2,7-octadièn-1-ol qui comprend la fourniture de 2,7-octadièn-1-ol et d'hydrogène à une zone de réaction en présence d'un catalyseur cuivre, le rapport molaire du 2,7-octadièn-1-ol sur l'hydrogène étant situé dans une plage allant de 99/1 à 75/25, et la réalisation de l'isomérisation en phase gazeuse.

2. Procédé selon la revendication 1, où le rapport molaire du 2,7-octadièn-1-ol sur l'hydrogène est situé dans la plage allant de 97/3 à 80/20.

3. Procédé selon l'une quelconque des revendications 1 ou 2, où le 2,7-octadièn-1-ol et l'hydrogène sont mélangés avec un gaz véhicule inerte au moment ou avant l'alimentation de la zone de réaction.

4. Procédé selon la revendication 3, dans lequel ledit gaz véhicule inerte est l'azote.

5. Procédé selon l'une quelconque des revendications 1 à 4, où ladite isomérisation est réalisée à une température située dans une plage allant de 100 à 260°C.

6. Procédé selon l'une quelconque des revendications 1 à 5, où ledit 2,7-octadièn-1-ol est fourni à la zone de réaction à une LHSV (vitesse spatiale horaire du liquide) de 0,01 à 20 h⁻¹.

7. Procédé selon l'une quelconque des revendications précédentes, où un alcool primaire ou secondaire, de formule (I)
R¹R²CH-OH (I)
où R¹ et R² indépendamment l'un de l'autre représentent un atome d'hydrogène ou un groupe alkyle, alcènyle, aryle ou cycloalkyle qui peut être substitué ; ou R¹ et R² ensemble représentent un groupe cycloalkyle, formé par la liaison de R¹ et R², qui peut être substitué, est fourni à la zone de réaction avec le 2,7-octadièn-1-ol.

8. Procédé selon la revendication 7, où R¹ et R² dans la formule (I), indépendamment l'un de l'autre, représentent un atome d'hydrogène, un alkyle linéaire ou ramifié ayant de 1 à 10 atomes de carbone ou un groupe alcènyle, aryle ou cycloalkyle qui peut être substitué par un groupe alkyle inférieur ayant 1 à 3 atomes de carbone ou un groupe phényle ; ou R¹ et R² ensemble représentent un groupe cycloalkyle qui peut être substitué par un groupe alkyle inférieur ayant de 1 à 3 groupes carbone.

9. Procédé selon la revendication 7 ou 8, où l'alcool est fourni à la zone de réaction avant ou en même temps que le 2,7-octadièn-1-ol.
